# EUROPÄISCHE PATENTANMELDUNG

(11) **EP 1 417 940 A1**
(43) Veröffentlichungstag der Anmeldung: **12.05.2004**
(21) Anmeldenummer: 02025113.8
(22) Anmeldetag: 08.11.2002
(51) Int. Cl.: A61F 2/44, A61F 2/30

(54) **Wirbelsäulenprothese**

(71) Anmelder: Waldemar Link (GmbH & Co.), 22339 Hamburg (DE)
(72) Erfinder: Keller, Arnold, D-23863 Kayhude (DE); McAfee,Dr. Paul C.,M.D. Scoliosis and Spine Center, Baltimore, MD 21204 (US)
(74) Vertreter: Glawe, Delfs, Moll & Partner

(57) **Zusammenfassung**

Wirbelsäulenprothese mit zwei entgegengesetzt gerichteten, äußeren Deckplatten (4, 18), die mit zwei Wirbelkörpern zu verbinden sind, und einem Überbrückungsteil (13) zur Überbrückung wenigstens eines zwischen den vorgenannten Wirbelkörpern befindlichen Wirbelkörpers. Zwischen dem Überbrükkungsteil (13) und wenigstens einer der beiden äußeren Deckplatten (4, 18) ist ein Gelenk (9, 11) zum Ersatz einer Bandscheibe vorgesehen.

## Beschreibung

Die Erfindung betrifft eine Wirbelsäulenprothese zur Überbrückung eines Wirbelkörpers. Eine bekannte Prothese dieser Art (WO 99/65412) weist zwei entgegengesetzt gerichtete, äußere Deckplatten auf, die mit den Deckplatten der Wirbelkörper zu verbinden sind, die dem zu überbrückenden Wirbelkörper benachbart sind. Verbunden werden sie in starrer Weise durch einen Überbrückungsteil, der in der Art einer Säule den zu überbrückenden Wirbelkörper ersetzt. Dies ergibt eine Versteifung, die häufig unerwünscht ist.

Die Erfindung findet eine stärkere Annäherung an die natürlichen Verhältnisse dadurch, daß zwischen dem Überbrückungsteil und wenigstens einer der beiden äußeren Deckplatten ein Gelenk zum Ersatz einer Bandscheibe vorgesehen ist. Vorzugsweise sind zwei solche Gelenke vorhanden, nämlich je eine zwischen dem Überbrückungsteil und einer der beiden äußeren Deckplatten. Zur Gestaltung der Gelenke kann auf sonstige Bandscheibenprothesen zurückgegriffen werden (EP-B-0 471 821, WO 00/53127, EP-A-0 298 233, FR-A-2 718 635, WO 01/01893).

Ein wichtiges Merkmal der Erfindung, das ggf. auch schutzunabhängig von den Merkmalen der Ansprüche 1 und 2 verdient, besteht darin, daß der zu überbrückende Wirbelkörper erhalten wird. Zu diesem Zweck ist der Überbrückungsteil mit einer Einrichtung zum Halten des zu überbrückenden Wirbelkörpers versehen und/oder bildet er zwischen zwei inneren Deckflächen einen Raum für die Aufnahme des zu überbrückenden Wirbelkörpers. Dieser kann dadurch von der Lastübertragung befreit werden, aber seine sonstigen Funktionen, die beispielsweise mit der Bildung der Facettengelenke und des Rückenmarkkanals verbunden sind, werden beibehalten. Die Einrichtung zum Halten des zu überbrückenden Wirbelkörpers und die dadurch bewirkte starre Verbindung zwischen dem Überbrückungsteil und dem Wirbelkörper stabilisiert nicht nur diesen Wirbelkörper, sondern kann auch umgekehrt dazu beitragen, daß der Wirbelkörper eine stabilisierende Wirkung auf die Prothese ausübt, sofern seine Facettengelenke dazu in der Lage sind.

Im einfachsten Fall ist die Einrichtung zum Halten des zu überbrückenden Wirbelkörpers in der Form von Schraubenlöchern ausgebildet, durch die hindurch mittels Knochenschrauben der zu überbrückende Wirbelkörper mit der Prothese verbunden wird. Vorzugsweise ist der Überbrückungsteil mit zwei inneren Deckflächen versehen, die zwischen sich einen Raum zur Aufnahme des zu überbrückenden Wirbelkörpers einschließen. Diese inneren Deckflächen können von inneren Deckplatten gebildet sein, die mit den äußeren Deckplatten je einen Bandscheibenersatzgelenk einschließen. Eine der inneren Deckflächen kann aber auch von der Innenseite einer äußeren Deckplatte gebildet sein, soweit dort kein Bandscheibenersatzgelenk vorgesehen ist.

Die inneren Deckflächen können starr miteinander verbunden sein und dadurch den zu überbrückenden Wirbelkörper von der Lastübertragung befreien. Beispielsweise können sie von einem und demselben Bauteil aus starrem Material (insbesondere Metall) gebildet sein. Bevorzugt wird jedoch eine Ausführung, in welcher sie von unterschiedlichen Teilen gebildet sind, die mit besonderen Verbindungseinrichtungen versehen sind, so daß der Abstand zwischen den inneren Deckflächen verstellt werden kann. Die Prothese kann dadurch der Höhe des zu ersetzenden Wirbelkörpers oder anderen individuellen Bedingungen angepaßt werden.

Damit die korrekte axiale Ausrichtung aller Prothesenteile trotz dieser Verstellbarkeit gewährleistet bleibt, sind die die inneren Endflächen bildenden Teile zweckmäßigerweise durch eine Parallelführung verbunden.

Die Erfindung wird im folgenden näher unter Bezugnahme auf die Zeichnung erläutert, die vorteilhafte Ausführungsbeispiele veranschaulicht. Es zeigen:
- Fig. 1: eine erste Ausführungsform der Prothese im Medianschnitt;
- Fig. 2: die mittlere Komponente der Prothese, ein wenig auseinandergezogen, im Medianschnitt;
- Fig. 3: einen Horizontalschnitt durch die Prothese gemäß Fig. 2; und
- Fig. 4 und 5: eine Seitenansicht und eine Ventralansicht einer dritten Ausführungsform der Prothese.

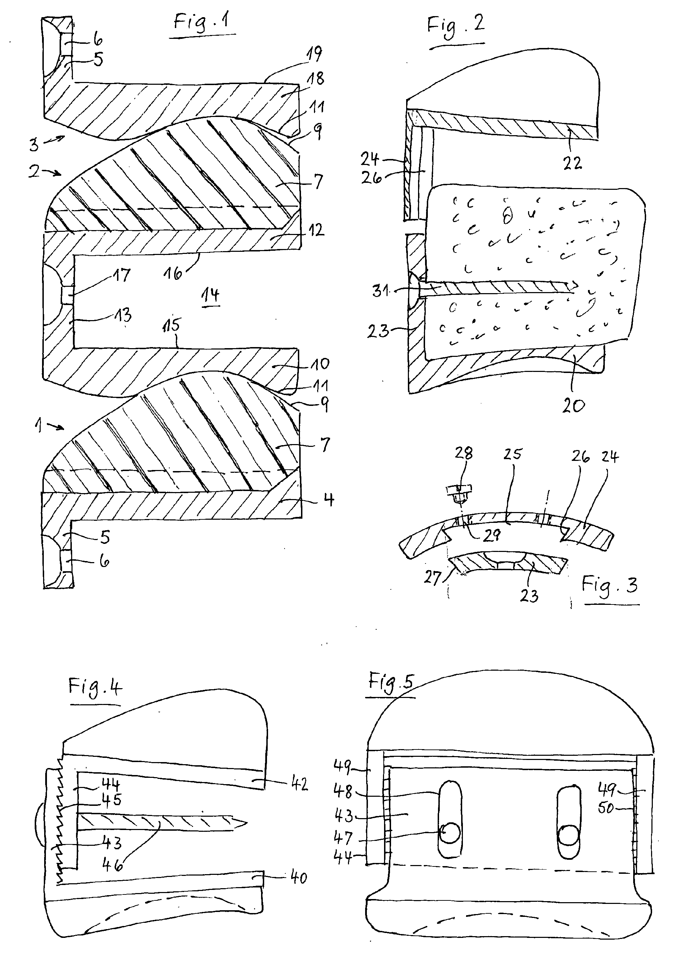

Die Prothese gemäß Fig. 1 umfaßt eine untere Komponente 1, eine mittlere Komponente 2 und eine obere Komponente 3. Diese Bezeichnungen beziehen sich auf die Anordnung in der Zeichnung. Sie können auch umgekehrt (die untere Komponente nach oben) implantiert werden.

Die untere Komponente 1 weist eine untere, äußere Deckplatte 4 auf, die in bekannter Weise mit einem unteren Wirbelkörper zu verbinden ist. Sie kann mit Einrichtungen versehen sein, die diese Verbindung sichern, beispielsweise einem Flansch 5 mit Schraubenloch 6. Auf der Oberseite der Deckplatte 4 ist ein Gleitkörper 7 aus einem gleitgünstigen Werkstoff, beispielsweise Polyethylen, befestigt. Zur Befestigung dient beispielsweise ein Paar von hinterschnittenen Randleisten, deren Oberkante bei 8 angedeutet ist. Oberseitig bildet der Gleitkörper 7 eine Gleitfläche 9.

Die mittlere Komponente 2 weist eine innere, untere Deckplatte 10 auf, deren Unterseite eine Gleitfläche 11 bildet, die komplementär der Gleitfläche 9 geformt ist und mit dieser ein Gelenk zum Ersatz einer Bandscheibe bildet. Die Gleitflächen 9, 11 sind beispielsweise sphärisch oder ellipsoidisch ausgebildet. Die innere, untere Deckplatte 10 besteht aus einem Werkstoff, der mit dem Material des Gleitkörpers 7 eine gleitgünstige Paarung bildet, vorzugsweise Metall wie Titan.

Die mittlere Komponente 2 weist ferner eine obere, innere Deckplatte 12 auf, die mit der inneren, unteren Deckplatte 10 durch einen Überbrückungsteil 13 starr verbunden ist.

Die beiden inneren Deckflächen 15, 16 an den inneren Deckplatten 10, 12 schließen einen Raum 14 zur Aufnahme eines mittleren Wirbelkörpers ein, der dem unteren, mit der Deckplatte 4 zu verbindenden Wirbelkörper benachbart ist. Da die inneren Deckplatten 10, 12 starr miteinander verbunden sind, ist auch die Größe des Aufnahmeraums 14 unveränderbar. Dies bietet sich für solche Fälle an, in denen der mittlere Wirbelkörper stark beschädigt ist. Nicht immer muß er fest mit der mittleren Komponente 2 der Prothese verbunden werden. Jedoch ist dies vorzuziehen. Zu diesem Zweck sind Schraubenlöcher 17 in dem Überbrückungsteil 13 vorgesehen. Wünscht man, daß der mittlere Wirbelkörper eng zwischen die inneren Deckflächen 15, 16 eingepaßt ist, so paßt man den Wirbelkörper dem Abstand der Flächen 15, 16 an oder wählt die mittlere Komponente 2 unter verschiedenen Größenstufen so aus, daß sie zu dem vorhandenen und ggf. angepaßten Wirbelkörper paßt.

Die innere, obere Deckplatte 12 trägt ebenso wie die äußere, untere Deckplatte 4 einen Gleitkörper 7 zur Bildung einer Gleitfläche 9, die mit der Gleitfläche 11 zusammenwirkt, die an der Unterseite einer äußeren, oberen Deckplatte 18 gebildet ist, deren Außenfläche 19 zur Verbindung mit einem oberen, dem mittleren Wirbelkörper benachbarten Wirbelkörper bestimmt ist. Sie kann ebenso wie die äußere, untere Deckplatte 4 mit einem ventralen Befestigungsflansch 5 mit Schraubenlöchern 6 versehen sein.

Die dargestellte Prothese enthält zwei Bändscheibenersatzgelenke unterhalb und oberhalb der mittleren Prothesenkomponente. Diese Ausführung bietet sich dann an, wenn der mittlere Wirbel und dessen Facettengelenke hinreichend erhalten sind für die Stabilisierung der mittleren Prothesenkomponente. Wenn diese Voraussetzung nur begrenzt erfüllt ist, kann man eines der beiden Bandscheibenersatzgelenke entfallen lassen und eine der beiden Deckplatten der mittleren Komponente unmittelbar oder über ein starres Anschlußstück mit dem unteren bzw. oberen Wirbelkörper verbinden.

Die zweite Ausführungsform der Prothese gemäß Fig. 2 und 3 gleicht derjenigen gemäß Fig. 1, soweit im folgenden nicht anderes beschrieben ist. Dargestellt ist lediglich die mittlere Komponente. Diese besteht aus zwei voneinander lösbaren Teilen, die je eine der Deckplatten 20, 22 bilden. Der Überbrückungsteil wird von zwei mit den Deckplatten 20, 22 starr verbundenen Flanschen 23, 24 gebildet, die mit komplementären, quer zur Ebene der Deckplatten 20, 22 verlaufenden Führungseinrichtungen versehen sind. Wie Fig. 3 zeigt, sind diese Führungseinrichtungen von einer Nut 25 in dem Flansch 24 gebildet, deren Flanken 26 schwalbenschwanzförmig hinterschnitten sind. Die Form der Nut 25 und ihrer Flanken 26 entspricht der Querschnittsform des Flansch 23 und seiner Ränder 27, so daß der Flansch 23 in der Nut 25 in Längsrichtung der Wirbelsäule verschiebbar geführt ist. Die gewählte Einstellung kann durch geeignete Verriegelungsmittel, beispielsweise ein Paar von Klemmschrauben 28 in Gewindelöchern 29 des Flanschs 24, gesichert werden. Die Einstellung wird in der Regel so gewählt, daß der mittlere Wirbelkörper 30 von den inneren Deckplatten 20, 22 eng und stabilisierend gefaßt ist, ohne aber an der Lastübertragung wesentlich beteiligt zu sein. In dem Raum zwischen den inneren Deckplatten 20, 22 wird er durch eine oder mehrere Knochenschrauben 31 gehalten. Auch die Deckplatten 20, 22 können mit geeigneten Haltemitteln (beispielsweise zahnartigen Vorsprüngen, die in den Wirbelkörper 30 eingreifen) versehen sein.

Zunächst wird die untere Deckplatte 20 mit dem Flansch 23 durch die Knochenschraube 31 am Wirbelkörper befestigt. Anschließend wird der Überbrückungsteil 24 über den Überbrükkungsteil 23 geschoben und mittels der Schrauben 28 fixiert.

Die in Fig. 4 und 5 dargestellte mittlere Komponente der dritten Ausführungsform weist ebenfalls zwei innere Deckplatten 40, 42 auf, die über Überbrückungsteile 43, 44 verstellbar und starr miteinander verbindbar sind. Die Flächen 45, an denen die Überbrückungsteile 43, 44 aneinander anliegen, sind mit einer Zahnung versehen, die dafür sorgt, daß die Überbrückungsteile 43, 44 in der einmal gewählten Raststellung sicher verbleiben, solange sie aneinander gehalten sind. Gehalten werden sie aneinander beispielsweise durch die Knochenschraube 46, die den mittleren Wirbelkörper im Raum zwischen den Deckplatten 40, 42 hält, oder durch besondere, nicht dargestellte Schrauben, die lediglich die Überbrükkungsteile 43, 44 zusammenhalten. Für die Schrauben sind in dem dem Knochen näher liegenden Überbrückungsteils 44 Durchgangslöcher oder Schraubenlöcher 47 vorgesehen, während in dem vom Knochen abgewandten Überbrückungsteil 43 Langlöcher 48 vorgesehen sind.

Der Überbrückungsteil 44 kann vorspringende Seitenleisten 49 aufweisen, zwischen denen die Ränder 50 des Überbrückungsteils 43 geführt sind, um dadurch eine parallele Lage der Deckplatten 40, 42 bei der Implantation zu gewährleisten.

Die Deckplatten 20, 22 bzw. 40, 42 können - wie in Fig. 2 und 4 gezeigt - entsprechend der Lordose der Wirbelsäule zueinander geneigt sein.

## Patentansprüche

1. Wirbelsäulenprothese mit zwei entgegengesetzt gerichteten, äußeren Deckplatten (4, 18), die mit zwei Wirbelkörpern zu verbinden sind, und einem Überbrückungsteil (13; 23, 24; 43, 44) zur Überbrückung wenigstens eines zwischen den vorgenannten Wirbelkörpern befindlichen Wirbelkörpers, **dadurch gekennzeichnet, daß** zwischen dem Überbrückungsteil (13; 23, 24; 43, 44) und wenigstens einer der beiden äußeren Deckplatten (4, 18) ein Gelenk (9, 11) zum Ersatz einer Bandscheibe vorgesehen ist.

2. Wirbelsäulenprothese nach Anspruch 1, **dadurch gekennzeichnet, daß** zwischen dem Überbrückungsteil (13; 23, 24; 43, 44) und beiden äußeren Deckplatten (4, 18) je ein Bandscheibenersatzgelenk (9, 11) angeordnet ist.

3. Wirbelsäulenprothese nach Anspruch 1 oder 2, **dadurch gekennzeichnet, daß** der Überbrückungsteil (13; 23, 24; 43, 44) eine Einrichtung (17) zum Halten des zu überbrückenden Wirbelkörpers (30) aufweist.

4. Wirbelsäulenprothese nach einem der Ansprüche 1 bis 3, **dadurch gekennzeichnet, daß** der Überbrückungsteil zwei innere Deckflächen (15, 16) bildet, zwischen denen sich ein Raum (14) zur Aufnahme des zu überbrückenden Wirbelkörpers (30) befindet.

5. Wirbelsäulenprothese nach Anspruch 4, **dadurch gekennzeichnet, daß** die inneren Deckflächen (15, 16) von inneren Deckplatten (10, 12; 20, 22; 40, 42) gebildet sind, die mit den äußeren Deckplatten (4, 18) je ein Bandscheibenersatzgelenk (9, 11) einschließen.

6. Wirbelsäulenprothese nach Anspruch 3 oder 4, **dadurch gekennzeichnet, daß** der Überbrückungsteil (13; 23, 24; 43, 44) die inneren Deckflächen (15, 16) starr miteinander verbindet.

7. Wirbelsäulenprothese nach Anspruch 6, **dadurch gekennzeichnet, daß** der Abstand der inneren Deckflächen (15, 16) voneinander einstellbar ist.

8. Wirbelsäulenprothese nach Anspruch 7, **dadurch gekennzeichnet, daß** die die inneren Deckflächen (15, 16) bildenden Teile durch eine Parallelführung (23-27; 49, 50) miteinander verbunden sind.
